# EUROPEAN PATENT APPLICATION

(11) **EP 4 154 999 A1**
(43) Date of publication of application: **29.03.2023**
(21) Application number: 21832405.1
(22) Date of filing: 30.06.2021
(51) Int. Cl.: B09B 3/00, C02F 3/28, C02F 11/00, C02F 11/04, C02F 11/08

(54) **WASTE TREATMENT SYSTEM AND WASTE TREATMENT METHOD**

(30) Priority: 02.07.2020 JP 2020115179
(71) Applicant: Mitsubishi Heavy Industries, Ltd., Tokyo 100-8332 (JP)
(72) Inventor: NOMA, Akira, Tokyo 100-8332 (JP); KAWAI, Kazuhiro, Tokyo 100-8332 (JP); FUJIKAWA, Keiji, Tokyo 100-8332 (JP); OKINO, Susumu, Tokyo 100-8332 (JP); NAKAGAWA, Yosuke, Tokyo 100-8332 (JP); FUKUNAGA, Koichi, Tokyo 100-8332 (JP); NAKAGAWA, Keiichi, Tokyo 100-8332 (JP); ADACHI, Haruka, Tokyo 100-8332 (JP); IKE, Takashi, Tokyo 100-8332 (JP); KAMITO, Ryo, Tokyo 100-8332 (JP); YAMADA, Masahiro, Tokyo 100-8332 (JP)
(74) Representative: Studio Torta S.p.A.
(86) International application number: PCT/JP2021/024811
(87) International publication number: WO 2022/004806

(57) **Abstract**

A waste treatment system includes: at least one reformer for hydrolyzing waste; and a microbial reactor for microbially degrading a reformed material containing at least a solid among the waste hydrolyzed in the at least one reformer.

## Description

### TECHNICAL FIELD

The present disclosure relates to a waste treatment system and a waste treatment method.

This application claims the priority of Japanese Patent Application No. 2020-115179 filed on July 2, 2020, the content of which is incorporated herein by reference.

### BACKGROUND

Patent Document 1 describes a treatment device for organic waste including organic wastewater or solid waste, for example, excess sludge from a sewage treatment plant, food waste such as kitchen waste, or livestock waste. In the treatment device, the organic waste is subjected to solid-liquid separation after being decomposed into soluble low-molecular-weight organic matter, the separated liquid is subjected to methane fermentation to produce biogas, and the separated solid is composted to generate fertilizer, thereby treating the organic waste.

### Citation List

### Patent Literature

Patent Document 1: JP4864339B

### SUMMARY

### Technical Problem

However, when treating waste with a lower moisture content than the organic waste described in Patent Document 1, such as municipal waste, even if the waste is hydrolyzed, most of objects to be treated are solids, and thus a generation amount of biogas is small in the treatment device of Patent Document 1. Further, although not only fertilizer but also fuel or the like can be produced from the separated solids, producing biogas is more profitable than producing the fertilizer or fuel, which may increase a waste treatment cost in the treatment device of Patent Document 1.

In view of the above, an object of at least one embodiment of the present disclosure is to provide a waste treatment system and a waste treatment method capable of decreasing a treatment cost of waste with low moisture content.

### Solution to Problem

In order to achieve the above object, a waste treatment system according to the present disclosure includes: at least one reformer for hydrolyzing waste; and a microbial reactor for microbially degrading a reformed material containing at least a solid among the waste hydrolyzed in the at least one reformer.

Further, a waste treatment method according to the present disclosure includes: a step of hydrolyzing waste; and a step of microbially degrading a reformed material containing at least a solid among the hydrolyzed waste.

### Advantageous Effects

According to a waste treatment system and a waste treatment method of the present disclosure, since valuables can be produced by microbially degrading hydrolyzed waste without solid-liquid separation, it is possible to treat even the waste with low moisture content at low cost.

### BRIEF DESCRIPTION OF DRAWINGS

FIG. 1 is a schematic configuration view of a waste treatment system according to Embodiment 1 of the present disclosure.
FIG. 2 is a schematic view showing an example of the configuration of a reformer in the waste treatment system according to Embodiment 1 of the present disclosure.
FIG. 3 is a schematic view showing another example of the configuration of the reformer in the waste treatment system according to Embodiment 1 of the present disclosure.
FIG. 4 is a schematic view showing still another example of the configuration of the reformer in the waste treatment system according to Embodiment 1 of the present disclosure.
FIG. 5 is a schematic configuration view of the waste treatment system according to Embodiment 2 of the present disclosure.
FIG. 6 is a schematic configuration view showing a modified example of the waste treatment system according to Embodiment 2 of the present disclosure.
FIG. 7 is a schematic configuration view of the waste treatment system according to Embodiment 3 of the present disclosure.
FIG. 8 is a graph showing an example of spectral data acquired by a near-infrared sensor in the waste treatment system according to Embodiment 3 of the present disclosure.
FIG. 9 is a graph showing spectral data of a plurality of reformed materials different in content of a reaction-unsuitable substance in the waste treatment system according to Embodiment 3 of the present disclosure.
FIG. 10 is a graph showing spectral data of some reformed materials different in concentration of the reaction-unsuitable substance in the waste treatment system according to Embodiment 3 of the present disclosure.
FIG. 11 shows a calibration curve for the concentration of the reaction-unsuitable substance in the reformed material used in the waste treatment system according to Embodiment 3 of the present disclosure.
FIG. 12 is a schematic configuration view of the waste treatment system according to Embodiment 4 of the present disclosure.
FIG. 13 is a schematic configuration view showing a part of a modified example of the waste treatment system according to Embodiment 4 of the present disclosure.
FIG. 14 is a schematic configuration view of the waste treatment system according to Embodiment 5 of the present disclosure.
FIG. 15 is a schematic configuration view of the waste treatment system according to Embodiment 6 of the present disclosure.
FIG. 16 is a schematic configuration view showing a modified example of the waste treatment system according to Embodiment 6 of the present disclosure.

### DETAILED DESCRIPTION

Hereinafter, a waste treatment system and a waste treatment method according to embodiments of the present disclosure will be described with reference to the drawings. The embodiments each indicate one aspect of the present disclosure, do not intend to limit the disclosure, and can optionally be modified within a scope of a technical idea of the present disclosure.

### (Embodiment 1)

### <Configuration of waste treatment system according to Embodiment 1 of present disclosure>

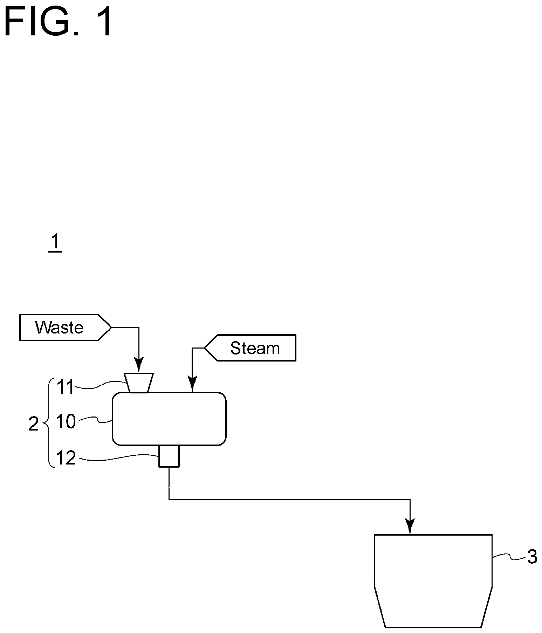

As shown in FIG. 1, a waste treatment system 1 according to Embodiment 1 of the present disclosure includes a reformer 2 for hydrolyzing waste such as municipal waste under coexistence of water or steam, and a microbial reactor 3 for microbially degrading a reformed material hydrolyzed in the reformer 2. The municipal waste, which is given as an example of waste, is characterized by mainly kitchen waste, paper waste, plastic waste, containing a small amount of metal, and having a relatively low moisture content. The waste to be treated by the waste treatment system 1 is not limited to municipal waste, but waste such as sludge generated by treating wastewater from a factory or the like, agricultural waste, etc. with a higher moisture content than the municipal waste can also be treated by the waste treatment system 1.

The reformer 2 is, for example, a reformer for receiving waste as it is from a vehicle, a plant, or the like where the waste is collected and hydrolyzes the waste in a batch manner with steam, and specifically, the reformer 2 is a batch-type reformer including a housing 10 with an input port 11 where the waste is input and a discharge port 12 where the reformed material is discharged. Opening and closing valves 18 and 19 are provided in the input port 11 and the discharge port 12, respectively, and the housing 10 can be sealed by closing the opening and closing valves 18, 19. The hydrolysis of the waste in the reformer 2 may be wet hydrolysis in which steam contacts the waste and heats the waste, or may be dry hydrolysis in which steam indirectly heats the waste without contacting the waste. In the case of dry hydrolysis, moisture in the waste within the housing 10 evaporates into water vapor, and the water vapor uniformly heats the waste within the housing 10. Further, moisture is necessary for hydrolysis, and moisture is supplied by causing moisture to adhere to the surface of the waste with the water vapor. Although one reformer 2 is shown in FIG. 1, a configuration where a plurality of reformers 2 are connected in series, a configuration where the plurality of reformers 2 are connected in parallel, or a combination of the configuration where the plurality of reformers 2 are connected in series and the configuration where the plurality of reformers 2 are connected in parallel may be adopted.

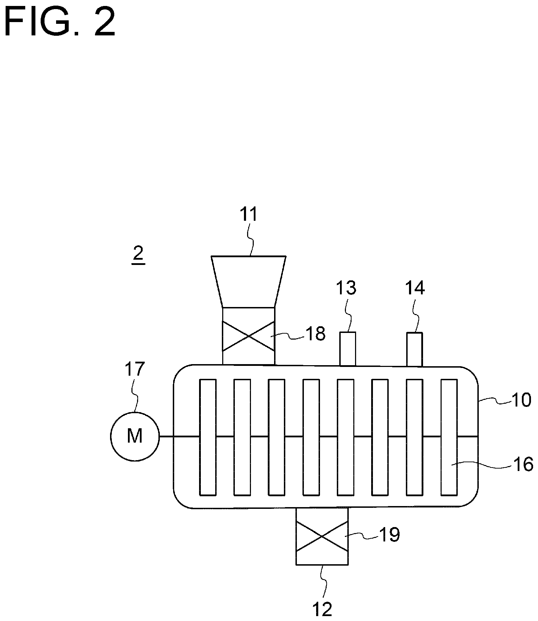

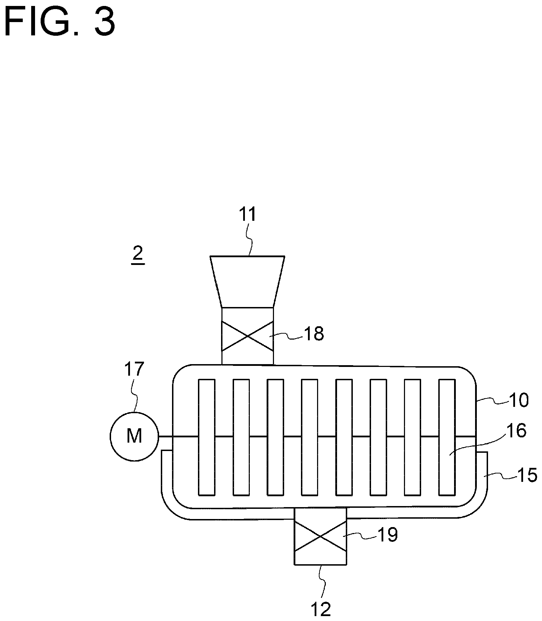

In the case of the reformer 2 performing wet hydrolysis, as shown in FIG. 2, a configuration can be adopted, as an example of the reformer 2, where the housing 10 includes at least one steam inlet 13 for causing steam to flow into the housing 10 and at least one purge nozzle 14 for purging a gas within the housing 10. Further, in the case of the reformer 2 performing dry hydrolysis, as shown in FIG. 3, a configuration can be adopted, as an example of the reformer 2, where the housing 10 includes a jacket 15 disposed so as to at least partially cover an outer surface of the housing 10. The jacket 15 constitutes a steam passage through which steam flows. The passage through which steam passes can also be disposed inside a reactor or on an agitating shaft. In the form of FIG. 3, the jacket 15 constitutes a heating part for hydrolyzing the waste within the housing 10 by heating moisture contained in the waste with heat of steam while avoiding contact with the waste. The heating part is not limited to the steam passage through which steam flows, but may be a part constituting a passage through which any heating medium such as a combustion exhaust gas flows, or a part such as an electric heater capable of indirectly heating the waste (especially moisture in the waste) without directly contacting the waste. In any form of the reformer 2, an agitatorl6 for agitating the waste within the housing 10 is provided inside the housing 10. The agitator 16 is driven by a motor 17.

As shown in FIG. 1, the configuration of the microbial reactor 3 is not particularly limited, but any configuration may be adopted as long as valuables are produced by using a biological action of microorganisms, with the reformed material obtained by hydrolyzing the waste in the reformer 2 being a raw material, and the configuration of the microbial reactor 3 may be, for example, a biogas fermentation tank for producing biogas such as methane as a valuable, a saccharification tank for producing sugar as a valuable from carbohydrates such as starch or cellulose, a composting device for producing compost by composting, or the like.

### <Operation of waste treatment system according to Embodiment 1 of present disclosure>

Next, the operation of the waste treatment system 1 according to Embodiment 1 of the present disclosure will be described. As shown in FIG. 1, the waste received in the housing 10 via the input port 11 is heated by steam while being agitated by the agitator 16 (see FIG. 2 or 3). Consequently, a hydrolysis reaction occurs in the waste. Conditions for this hydrolysis are not particularly limited, but can be set, for example, such that a cell fluid flows out from a cell contained in kitchen waste and as such conditions, it is possible to use temperatures between normal temperature and approximately 250°C and pressures between atmospheric pressure and approximately 40 atmospheres.

The kitchen waste in the waste mainly contains proteins, carbohydrates, fats, and by hydrolyzing the kitchen waste, pinholes are formed in a cell membrane and a cell wall, and the cell membrane and the cell wall are dissolved, resulting in outflow of the cell fluid. Consequently, the kitchen waste is made finer, and a polymer component is subjected to degradation. In addition, volatile fatty acid (VFA) such as acetic acid is increased.

Hydrophobic lignin or hemicellulose forming a plant such as wood in the waste is converted to a hydrophilic substance and dissolved by hydrolysis, thereby exposing cellulose. Paper waste in the waste becomes hydrophilic due to dissolution of chemical on a surface. Further, by being agitated with the agitator 16, the paper waste is finely pulverized, softened, and decreased in diameter. Plastic waste in the waste is heated and softened, and is sheared by the agitation with the agitator 16 and decreased in diameter.

The reformed material, which is the waste hydrolyzed in the reformer 2, includes each of generated components generated as described above from the kitchen waste, the paper waste (including wood, etc.), the plastic waste, and a small amount of metal hardly affected by hydrolysis. Due to the relatively low moisture content of the waste having the above-described composition, the composition of the reformed material is mostly solid component with very little liquid. Such reformed material is flowed out from the housing 10 via the discharge port 12 and transferred to the microbial reactor 3. If the waste has a high moisture content, such as sludge, the amount of liquid in the reformed material also increases, resulting in a slurry reformed material. Even in such a case, the entire amount of the reformed material is transferred to the microbial reactor 3 without the reformed material undergoing solid-liquid separation. In the microbial reactor 3, the reformed material is degraded by being subjected to the biological action of microorganisms, producing valuables.

The kitchen waste in the waste is made finer by hydrolysis, increasing the surface area of a kitchen waste-derived component. Since the area subjected to the biological action of microorganisms increases, the degradation is promoted. If uneven distribution of the kitchen waste-derived components is suppressed and homogenized by making the kitchen waste finer, it is possible to homogenize the activity of the biological action, and the degradation is stabilized. In addition, the increase in VFA promotes the degradation. Further, by degrading the kitchen waste-derived component, fat bubbling in the microbial reactor 3 is suppressed. The occurrence of such bubbling causes a trouble in which an overflow port (not shown) of the microbial reactor 3 is clogged, but it is possible to suppress the occurrence of such trouble.

Since cellulose of the paper waste or plant in the waste is exposed by hydrolysis, microorganisms can easily access the cellulose, promoting the degradation. Further, since these components become hydrophilic and are decreased in diameter, these components do not float in the microbial reactor 3, making it possible to reduce a risk of inhibiting the degradation. Since the plastic waste in the waste is also decreased in diameter by hydrolysis, it is possible to reduce the risk of inhibiting the degradation.

Thus, since the valuables can be produced by degrading the reformed material, which is obtained by hydrolyzing the waste in the reformer 2, in the microbial reactor 3 without solid-liquid separation, it is possible to produce the valuables even from the waste with low moisture content. Further, since the waste treatment system 1 does not require a device for solid-liquid separation of the reformed material and produces only the valuables with a high unit price such as biogas, it is possible to treat the waste at lower cost, compared to a system for performing solid-liquid separation on the reformed material, producing biogas from the liquid which has undergone solid-liquid separation, and producing fuel, fertilizer, etc. from the solid which has undergone solid-liquid separation.

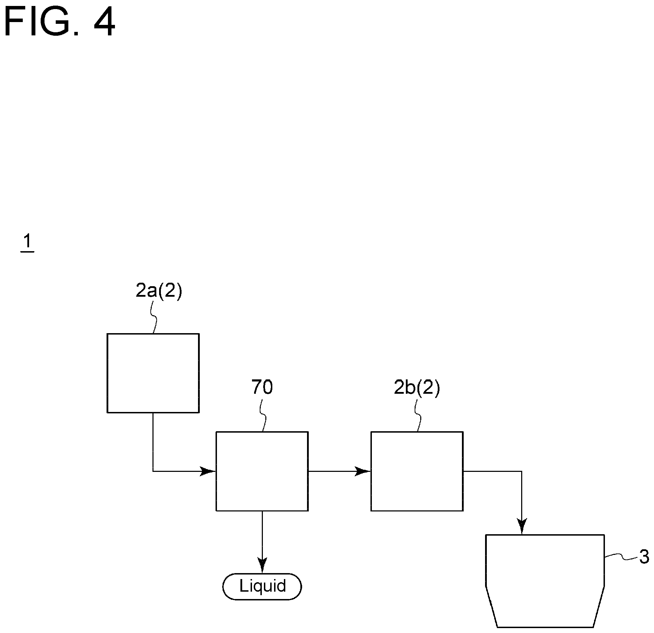

As shown in FIG. 4, in Embodiment 1, if two reformers 2 (a first reformer 2a and a second reformer 2b) connected in series are provided, a solid-liquid separator 70 is provided between the first reformer 2a and the second reformer 2b, and solid-liquid separation may be performed on the reformed material of the first reformer 2a to transfer only the solid obtained by solid-liquid separation to the second reformer 2b. By solid-liquid separation, a nitrogen compound, such as protein, that causes production of melanoidin can be separated to a liquid side, making it possible to suppress production of melanoidin in hydrolysis in the second reformer. Inflow of melanoidin into a methane fermentation tank serving as the microbial reactor 3 inhibits methane fermentation. Thus, by performing solid-liquid separation on the reformed material of the first reformer to transfer only the solid obtained by solid-liquid separation to the second reformer, it is possible to reduce a risk of inhibiting methane fermentation in the methane fermentation tank. Since the liquid obtained by solid-liquid separation contains the nitrogen compound, the liquid may be supplied to the methane fermentation tank by bypassing the second reformer 2b. Whereby, the nitrogen component can be replenished to the methane fermentation tank.

In Embodiment 1, the steam used for heating the waste in the reformer 2 may be supplied to the microbial reactor 3 to be used as a heat source for heat retention during microbial reaction in the microbial reactor 3. Whereby, an operating cost can be reduced compared to a case where a heat source necessary for the operation of the microbial reactor 3 is separately prepared.

### (Embodiment 2)

Next, the waste treatment system according to Embodiment 2 will be described. The waste treatment system according to Embodiment 2 is obtained by adding, to Embodiment 1, a separator for separating, from the reformed material, a reaction-unsuitable substance which does not contribute to the microbial degradation in the microbial reactor 3, that is, which is unsuitable for the degradation. In Embodiment 2, the same constituent elements as those in Embodiment 1 are associated with the same reference characters and not described again in detail.

### <Configuration of waste treatment system according to Embodiment 2 of present disclosure>

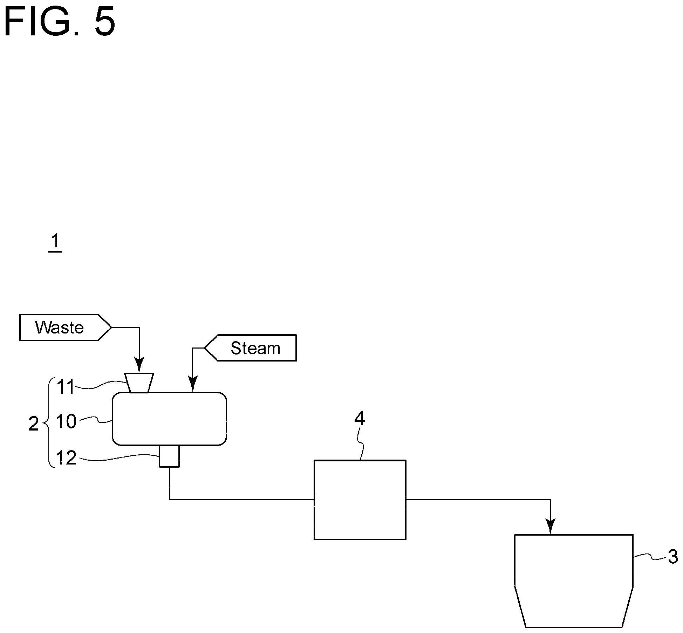

As shown in FIG. 5, the waste treatment system 1 according to Embodiment 2 of the present disclosure includes a separator 4 disposed between the reformer 2 and the microbial reactor 3. The separator 4 is for separating the reformed material into a large particle size component and a small particle size component having a smaller particle size than the large particle size component. The separator 4 is, for example, a screen having any mesh size, and the mesh size corresponds to a particle size at a boundary between the large particle size component and the small particle size component. Other configurations are the same as those in Embodiment 1.

### <Operation of waste treatment system according to Embodiment 2 of present disclosure>

In the waste treatment system 1 according to Embodiment 2 of the present disclosure, the reformed material is separated into the large particle size component and the small particle size component in the separator 4, only the small particle size component is supplied to the microbial reactor 3, and only the small particle size component is degraded to produce valuables. A principal component of the large particle size component is a component that has a relatively large particle size even after hydrolysis in the reformer 2, and cannot be degraded in the microbial reactor 3, such as those derived from plastic waste or metal. To put it another way, the large particle size component and the small particle size component are, respectively, a reaction-unsuitable substance and a reaction-suitable substance for microbial reaction.

In Embodiment 2, since such large particle size component is separated from the reformed material by the separator 4 and only the small particle size component is supplied to the microbial reactor 3, it is possible to reduce the amount of the reaction-unsuitable substance supplied to the microbial reactor 3. As a result, it is possible to reduce the risk of inhibiting the degradation in the microbial reactor 3, and to efficiently perform the degradation.

Regarding the screen as an example of the separator 4, waste with a total solid concentration (TS) of 53% is hydrolyzed (heated to predetermined temperatures between normal temperature and 240°C while being agitated in a closed container, and held for a certain period of time), and then a reformed material of the waste is passed through the screen including a mesh. As a result, relative to the recovery rate of 40 wt% in a case where the reaction-suitable substance (an organic matter such as paper, kitchen waste, etc.) as the small particle size component is crushed as it is in the conventional manner and separated with the screen, the recovery rate of 80 wt% can be obtained by using the method of the present invention, and it is possible to reduce the mixing ratio of the reaction-unsuitable substance (plastic waste, etc.) in the reaction-suitable substance to not greater than 10 wt%. Thus, since it is found that by using the screen, it is possible to remove, to a certain extent, the reaction-unsuitable substance from the reformed material having undergone hydrolysis, by providing the separator 4 between the reformer 2 and the microbial reactor 3, it can be presumed that the risk of inhibiting the degradation in the microbial reactor 3 can be reduced and the degradation can efficiently be performed. In the present embodiment, the example of the separation based on the difference in particle size is shown. However, as means for the separation, specific gravity separation, wind separation, wet separation, etc. can also be used. Thus, the separator 4 can also be a device using specific gravity separation, wind separation, wet separation, etc., or a combination thereof. If the microbial reactor 3 is a methane fermentation tank, wet separation is preferable because of the necessity of replenishing moisture, and if the microbial reactor 3 is a composting device, wind separation or screen separation is preferable because of the necessity of having a low moisture content. An air jet spray or the like may be used to improve the recovery rate of the small particle size component.

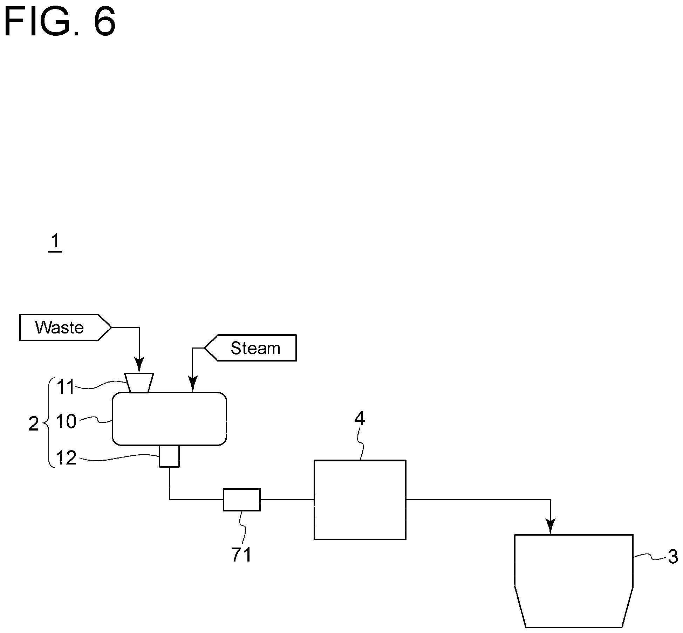

In Embodiment 2, when treating something like sludge with a relatively high moisture content, since the TS of the reformed material flowing out from the reformer 2 is low, the reaction-unsuitable substance may not successfully be separated in the separator 4. In such a case, after hydrolysis is performed in the reformer 2, the purge nozzle 14 (see FIG. 2) is opened for a certain period of time to perform the same operation as vacuum drying, making it also possible to reduce the moisture content of the reformed material and adjust the TS of the reformed material. In this case, the purge nozzle 14 constitutes a moisture adjusting device for adjusting the moisture content of the reformed material. Regarding this form, as shown in FIG. 6, a drying device 71 of any configuration, which serves as the moisture adjusting device capable of adjusting the moisture content of the reformed material, may be disposed on a reformed material transfer line 5 through which the reformer 2 and the separator 4 communicate with each other. In this case, a dehydrator may be provided as the moisture adjusting device. Conversely, in order to increase the fluidity of the hydrolyzed reformed material, the moisture adjusting device can also adjust the TS of the reformed material by adding moisture to the reformed material to increase the moisture content. In this case, a water spray may be provided as the moisture adjusting device. Further, if the moisture adjusting device is configured such that moisture is added to the small particle size component separated in the separator 4 and excess moisture is removed, the moisture adjusting device can also function as a washing device for washing the reaction-suitable substance adhering to the reaction-suitable substance. Thus, the reaction-suitable substance can be supplied to the microbial reactor 3 after the content of a fermentation inhibitor such as melanoidin in the reaction-suitable substance is reduced, making it possible to reduce the risk of inhibiting the reaction in the microbial reactor 3. Further, if the moisture adjusting device is used as a washing device for washing the reaction-unsuitable substance which is the large particle size component separated in the separator 4, it is possible to recover the small particle size component adhering to the reaction-unsuitable substance as the reaction-suitable substance, and it is possible to improve the recovery rate of the reaction-suitable substance. The moisture adjusting device can also be disposed inside the separator 4. The reformed material transfer line 5 may be a pipe if the reformed material is in the form of slurry, or may be a conveyor or the like if the reformed material is solid. Even if the reformed material is solid, as long as the reformed material can be pumped by air or the like, the reformed material transfer line 5 may be the pipe.

### (Embodiment 3)

Next, the waste treatment system according to Embodiment 3 will be described. Relative to Embodiment 2, the waste treatment system according to Embodiment 3 is configured to estimate the content of the reaction-unsuitable substance in the reformed material. In Embodiment 3, the same constituent elements as those in Embodiment 2 are associated with the same reference characters and not described again in detail.

### <Configuration of waste treatment system according to Embodiment 3 of present disclosure>

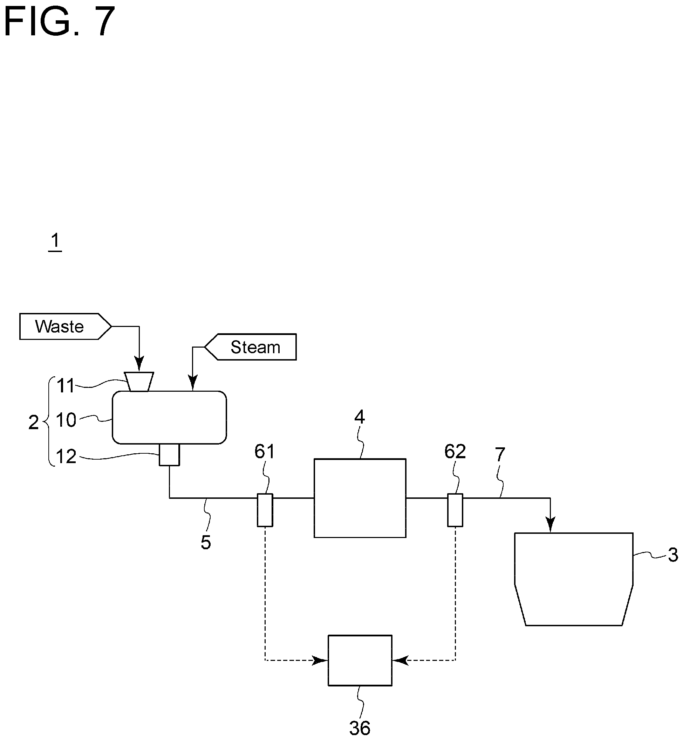

As shown in FIG. 7, in Embodiment 3, near-infrared sensors 61, 62, such as hyperspectral cameras, are respectively disposed on the reformed material transfer line 5 and a reformed material transfer line 7 through which the separator 4 and the microbial reactor 3 communicate with each other. The near-infrared sensors 61, 62 are electrically connected to a control device 36. The specific configuration of the reformed material transfer line 7 is the same as that of the reformed material transfer line 5. Other configurations are the same as those in Embodiment 2.

### <Operation of waste treatment system according to Embodiment 3 of present disclosure>

The operation in Embodiment 3 is basically the same as that in Embodiment 2. Embodiment 3 is different from Embodiment 2 in that the near-infrared sensor 61, 62 acquires spectral data of the reformed material while the reformed material is transferred from the reformer 2 to the microbial reactor 3, the acquired spectral data is transmitted to the control device 36, and the control device 36 performs an operation of estimating the content of the reaction-unsuitable substance in the reformed material based on the spectrum data. Operations different from those of Embodiment 2 will be described below.

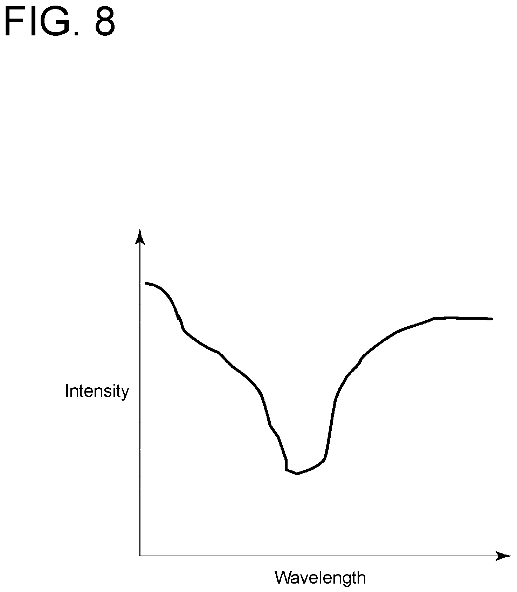

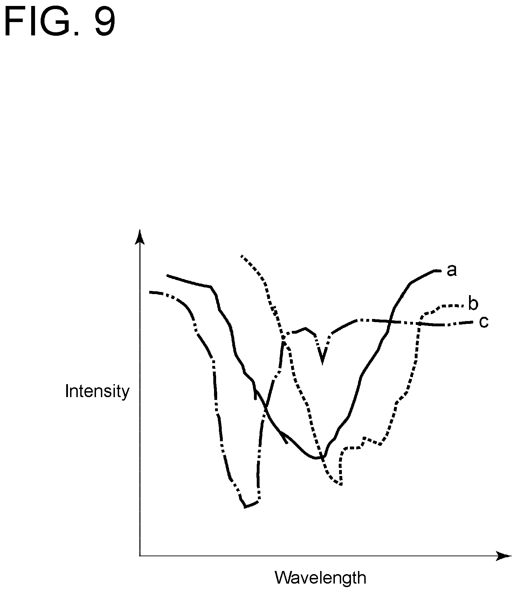

FIG. 8 shows an example of the spectral data acquired by the near-infrared sensor 61, 62. As indicated by a, b, c (not limited to three, but may be not less than four) in FIG. 9, the control device 36 saves in advance as a database spectral data of the reformed material different in content of the reaction-unsuitable substance. The control device 36 identifies, by using means such as the weighted average method or the kernel method, the acquired spectral data (FIG. 8) by combining a plurality of spectral data saved as the database, and can estimate the concentration of the reaction-unsuitable substance based on the weighted value.

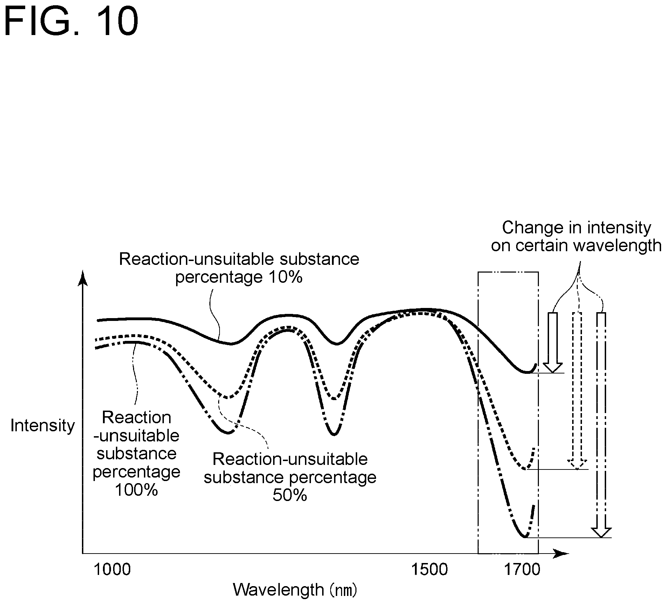

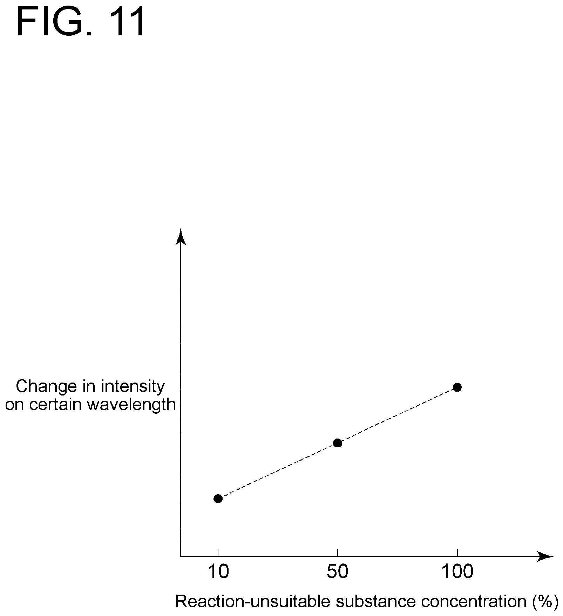

As another example, for example, as shown in FIG. 10, spectral data of several reformed materials different in concentration of the reaction-unsuitable substance (10%, 50%, 100% in FIG. 10, but these are merely examples) is acquired, a change in intensity between two different specific wavelengths (for example, 1,500 nm and 1,700 nm) in each spectrum data is read, and a relationship (calibration curve) between the concentration of the reaction-unsuitable substance and the change in intensity between the two different specific wavelengths is created in advance as shown in FIG. 11 and is stored in the control device 36. The control device 36 can read the absorbance at the specific wavelength of the spectral data acquired by the near-infrared sensor 61, 62, and estimate the concentration of the reaction-unsuitable substance in the reformed material based on this calibration curve.

As still another example, in addition to the near-infrared sensor 61, 62, for example, a piezoelectric sensor or the like is provided to measure the weight of the reformed material being transferred. It is also possible to estimate the type of reaction-unsuitable substance from the spectral data acquired by using the near-infrared sensor 61, 62, and thus it is also possible to estimate, from the type of reaction-unsuitable substance and the weight of the reformed material, the weight ratio of the reaction-unsuitable substance and the reaction-suitable substance in the reformed material being transferred.

Based on the content of the reaction-unsuitable substance in the reformed material thus estimated, it is possible to detect abnormalities in the reformer 2 and the separator 4 or abnormality in the waste input to the reformer 2.

In Embodiment 3, the content of the reaction-unsuitable substance in the reformed material is estimated by the near-infrared sensor 61, 62. However, the present invention is not limited to this mode. With the near-infrared sensor, it is also possible to predict the property of the waste input to the reformer 2. For example, the near-infrared sensor acquires spectral data of the waste, and from the acquired spectral data, it is possible to grasp the percentage of moisture/protein/carbohydrate/fat/plastic components, etc. Based on the property of the waste thus acquired, it is possible to set hydrolysis conditions (temperature/pressure/time/agitation speed, etc.) in the reformer 2. Further, it is also possible to set separation conditions (mesh size, water content, frequency, etc.) in the separator 4. A digital camera may be used instead of the near-infrared sensor 61, 62, and the property of the waste may be predicted based on an image taken by the digital camera.

If the property of the waste is known in advance for reasons that, for example, a waste receiving route is clear, the hydrolysis conditions in the reformer 2 or the separation conditions in the separator 4 can be set based on the property. In addition, the property of the waste can be estimated with, for example, AI prediction using information about the property of the waste such as waste collection date, event calendar (information about the type of waste), past analysis result of waste, local purchasing information or the like. That is, the control device 36 detects, as a detection device, the information about the property of the waste as an indicator of a hydrolysis state of the waste in the reformer 2, and then the control device 36 may estimate the hydrolysis state based on this indicator.

### (Embodiment 4)

Next, the waste treatment system according to Embodiment 4 will be described. Relative to any of Embodiments 1 to 3, the waste treatment system according to Embodiment 4 is configured to adjust the hydrolysis conditions based on the hydrolysis state of the waste in the reformer 2. Embodiment 4 will be described below with a configuration where the hydrolysis conditions are adjusted, relative to Embodiment 2. However, Embodiment 4 may be configured to adjust the hydrolysis conditions, relative to Embodiment 1 or 3. In Embodiment 4, the same constituent elements as those in Embodiment 2 are associated with the same reference characters and not described again in detail.

### <Configuration of waste treatment system according to Embodiment 4 of present disclosure>

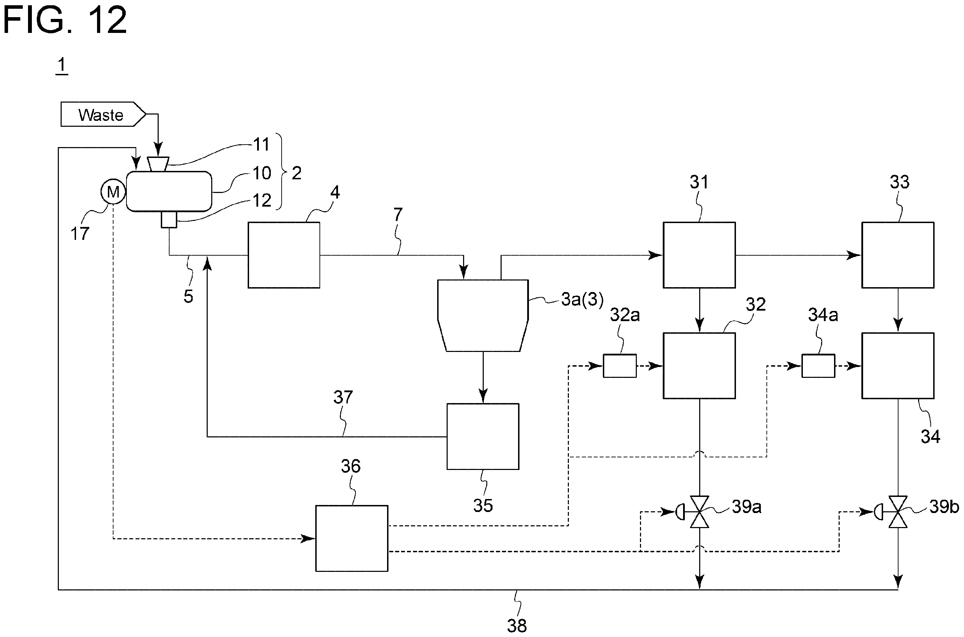

In Embodiment 4, the microbial reactor 3 will be described as a biogas fermentation tank 3a for producing biogas such as methane. As shown in FIG. 12, the waste treatment system 1 according to Embodiment 4 of the present disclosure includes a gas holder 31 for storing biogas produced in the biogas fermentation tank 3a, a combustion boiler 32 for generating steam by using the biogas stored in the gas holder 31 as fuel, a gas engine 33 driven with the biogas stored in the gas holder 31 as fuel, an exhaust gas boiler 34 for generating steam by heat of an exhaust gas from the gas engine 33, and a dehydrator 35 for dehydrating a fermentation residue in the biogas fermentation tank 3a. Although not an essential configuration, a water injection pipe 37 may be provided through which the dehydrator 35 and the reformed material transfer line 5 communicate with each other.

Each of the combustion boiler 32 and the exhaust gas boiler 34 communicates with the steam inlet 13 (see FIG. 2) or the jacket 15 (see FIG. 3) of the reformer 2 through a steam supply pipe 38, and it is configured such that steam generated in each of the combustion boiler 32 and the exhaust gas boiler 34 is supplied to the reformer 2. The steam supply pipe 38 is provided with steam supply amount adjustment valve 39a, 39b for adjusting the amount of steam supplied to the reformer 2 from each of the combustion boiler 32 and the exhaust gas boiler 34. The waste treatment system 1 includes a combustion boiler controller 32a for controlling a temperature and a generation amount of steam generated in the combustion boiler 32, and an exhaust gas boiler controller 34a for controlling a temperature and a generation amount of steam generated in the exhaust gas boiler 34. Further, the waste treatment system 1 includes the control device 36, and the control device 36 is electrically connected to each of the motor 17 for driving the agitator 16 of the reformer 2, the combustion boiler controller 32a, the exhaust gas boiler controller 34a, and the steam supply amount adjustment valve 39a, 39b. Although not illustrated in FIG. 12, the control device 36 is configured to acquire the hydrolysis state (temperature, pressure, etc.) in the reformer 2.

The combustion boiler controller 32a and the exhaust gas boiler controller 34a control the temperature and the generation amount of steam generated in the combustion boiler 32 and the exhaust gas boiler 34, respectively, and the steam supply amount adjustment valves 39a, 39b adjust the amount of steam supplied to the reformer 2. Then, since these constituent elements adjust the hydrolysis conditions (temperature, pressure, etc.) of the waste in the reformer 2, these constituent elements each constitute the adjustment device for adjusting the hydrolysis conditions of the waste in the reformer 2. Other configurations are the same as those in Embodiment 2.

### <Operation of waste treatment system according to Embodiment 4 of present disclosure>

From the operation of hydrolyzing the waste in the reformer 2 to the operation of producing biogas as a valuable in the microbial reactor 3, that is, the biogas fermentation tank 3a, are the same as those in Embodiment 2. The biogas produced in biogas fermentation tank 3a is stored in the gas holder 31. Biogas is supplied from the gas holder 31 to each of the combustion boiler 32 and the gas engine 33, according to operating conditions of the combustion boiler 32 and the gas engine 33. In the combustion boiler 32, steam is generated by using biogas as fuel. In the exhaust gas boiler 34, steam is generated by heat of the exhaust gas from the gas engine. Steam generated in each of the combustion boiler 32 and the exhaust gas boiler 34 is supplied to the reformer 2 and used to hydrolyze the waste.

Although an operation method for each of the combustion boiler 32 and the exhaust gas boiler 34 is not particularly limited, an example of the operation method will be described. While the waste is hydrolyzed in the reformer 2, the exhaust gas boiler 34 is always operated. While the hydrolysis conditions in the reformer 2 can be adjusted only by the steam generated in the exhaust gas boiler 34, the combustion boiler 32 is not operated. When the hydrolysis conditions are adjusted according to the amount of the waste input to the reformer 2 or the composition of the waste component, if the steam generated in the exhaust gas boiler 34 alone is insufficient, the combustion boiler 32 is operated to supply not only the steam generated in the exhaust gas boiler 34 but also the steam generated in the combustion boiler 32 to the reformer 2. Thus, it is possible to widen an adjustment range of the hydrolysis conditions in the reformer 2, compared to the case where only the exhaust gas boiler 34 is provided. It is possible to further widen the adjustment range of the hydrolysis conditions in the reformer 2 by providing not less than two gas engines 33 and operating the number of gas engines 33 appropriate to the amount of the steam generated in the exhaust gas boiler 34.

In order to adjust the hydrolysis conditions in the reformer 2, it is necessary to detect the hydrolysis state in the reformer 2. Although a method for detecting the hydrolysis state is not particularly limited, an example of the method will be described. The control device 36 detects a torque of the motor 17 as an indicator of the hydrolysis state of the waste in the reformer 2. The torque of the motor 17 fluctuates according to the progress of hydrolysis which is started after the waste is input to the reformer 2. It is possible to detect the hydrolysis state from the fluctuation in torque of the motor 17. In addition, it is possible to detect the hydrolysis state from indicators that consider various measurement values such as a hydrolysis temperature, pressure, and time, a steam supply amount, etc.

A temporal change in torque of the motor 17 can vary depending on the composition of the waste or the like. However, if there is no significant difference in composition of the waste hydrolyzed each time in the reformer 2, such temporal change in torque of the motor 17 is detected in advance, and by being contrasted with the temporal change detected in advance, it is possible to estimate the hydrolysis state in the reformer 2. Thus, the control device 36 for detecting the torque of the motor 17 constitutes a detection device for detecting the indicator of the hydrolysis state of the waste in the reformer 2. It may be configured such that a detection device for detecting the torque of the motor 17 is provided separately from the control device 36, and the control device 36 receives a signal regarding the torque of the motor 17 from this detection device.

As a result of detecting the hydrolysis state based on the torque of the motor 17, it is conceivable that, for example, the waste has not been made finer, the waste has been made too finer and the plastic waste has been made finer to a particle size which is too small to be achieved under normal circumstances, or the like. In the former case, for example, it is conceivable to increase the amount of steam supplied to the reformer 2 because it is necessary to increase the hydrolysis temperature, and in the latter case, for example, it is conceivable to decrease the amount of steam supplied to the reformer 2 because the hydrolysis temperature may be lowered from the viewpoint of the operating cost of the waste treatment system 1.

The control device 36 thus estimates the hydrolysis state in the reformer 2 based on the torque of the motor 17, and based on the estimated hydrolysis state, controls the adjustment device, that is, at least one of the combustion boiler controller 32a, the exhaust gas boiler controller 34a, or the steam supply amount adjustment valve 39a, 39b to adjust the temperature (pressure) or the supply amount of steam supplied to the reformer 2. As a result, the hydrolysis state in the reformer 2 changes. In this manner, the control device 36 adjusts the hydrolysis conditions in the reformer 2 based on the hydrolysis state in the reformer 2. Further, the noncompletion/completion of hydrolysis can be known from such hydrolysis state, and thus it is also possible to adjust the hydrolysis time.

Thus, in Embodiment 4, since the reformed material on the conditions suitable for microbial reaction can be supplied to the biogas fermentation tank 3a, it is possible to efficiently produce biogas by microbial reaction.

In Embodiment 2, it is described that the TS of the reformed material may be adjusted to increase the moisture content by adding moisture to the reformed material to increase the fluidity of the hydrolyzed reformed material. In order to perform such adjustment on the TS of the reformed material in Embodiment 4, boiler blow water or water dehydrated from the fermentation residue of the biogas fermentation tank 3a in the dehydrator 35 may be supplied to the reformed material in the reformed material transfer line 5 via the water injection pipe 37. Thus, the water injection pipe 37 constitutes the moisture adjusting device. Since the dehydrated water or the boiler blow water contains ammonia, the reformed material can also be replenished with a nitrogen-containing substance if the nitrogen content in the waste is low. From the viewpoint of replenishment of the nitrogen-containing substance, the dehydrated water or the boiler blow water is not limited to being supplied to the reformed material in the reformed material transfer line 5, but may be supplied to the biogas fermentation tank 3a or the reformer 2. In this case, the water injection pipe 37 may be connected to the biogas fermentation tank 3a or the reformer 2. Moreover, in Embodiment 3, the control device 36 estimates the hydrolysis conditions based on the torque of the motor 17. However, the control device 36 may estimate the hydrolysis conditions based on a current value of the motor 17. That is, the current value of the motor 17 may be used as an indicator of the hydrolysis state of the waste in the reformer 2.

### <Modified example of waste treatment system according to Embodiment 4 of present disclosure>

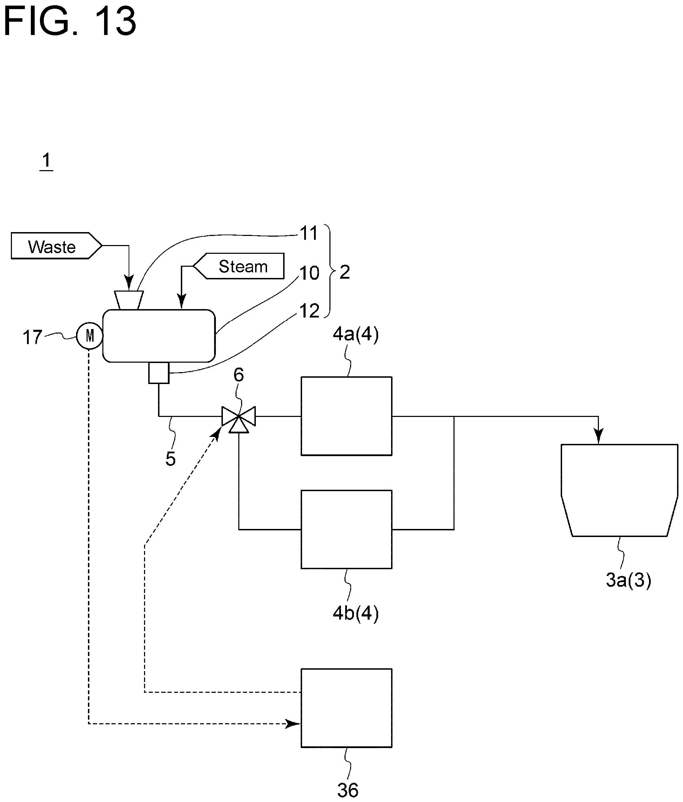

In Embodiment 4, the hydrolysis conditions in the reformer 2 are adjusted based on the hydrolysis state in the reformer 2. However, the present invention is not limited to this mode. For example, as shown in FIG. 13, as the separator 4, two separators 4a and 4b different in mesh size may be disposed to be arranged in parallel to the flow of the reformed material. In this case, the mesh size of the separator 4a is smaller than that of the separator 4b. The reformed material transfer line 5 is provided with a switching device 6 for supplying the reformed material to either one of the separators 4a and 4b. The motor 17 and the switching device 6 are electrically connected to the control device 36.

If irregular fluctuations appear without periodic increases and decreases appearing in the torque of the motor 17 even after the start of hydrolysis and it is considered that the waste has not been made finer, the control device 36 operates the switching device 6 to supply the reformed material to the separator 4b. The size of the reaction-unsuitable substance such as plastic waste in particular remains large unless the waste has been made finer, the separator 4b having the large mesh size is selected to remove the reaction-unsuitable substance, and even the reaction-unsuitable substance large in size can be supplied to the biogas fermentation tank 3a. On the other hand, if the periodic increase and decrease range is small and it is considered that the waste has sufficiently been made finer, the control device 36 operates the switching device 6 to supply the reformed material to the separator 4a. Thus, it is possible to also remove the reaction-unsuitable substance that has been made finer.

In the modified example of Embodiment 4 shown in FIG. 13, the two separators 4a, 4b different in mesh size are provided. However, the number of separators is not limited to two, but not less than three separators different in mesh size may be provided. Thus, not less than three separators different in mesh size can be selected according to the hydrolysis conditions in the reformer 2.

### (Embodiment 5)

Next, the waste treatment system according to Embodiment 5 will be described. The waste treatment system according to Embodiment 5 is obtained by modifying any of Embodiments 1 to 4 such that the hydrolysis state of the waste in the reformer 2 is estimated based on the reaction conditions in the microbial reactor 3 and based on the estimated hydrolysis state, the hydrolysis conditions of a next batch of waste in the reformer 2 are adjusted. Hereinafter, a configuration obtained by introducing the above-described modification to Embodiment 4 will be described. However, Embodiment 5 may be configured by introducing the above-described modification to any of Embodiments 1 to 3. In Embodiment 5, the same constituent elements as those in Embodiment 4 are associated with the same reference characters and not described again in detail.

### <Configuration of waste treatment system according to Embodiment 5 of present disclosure>

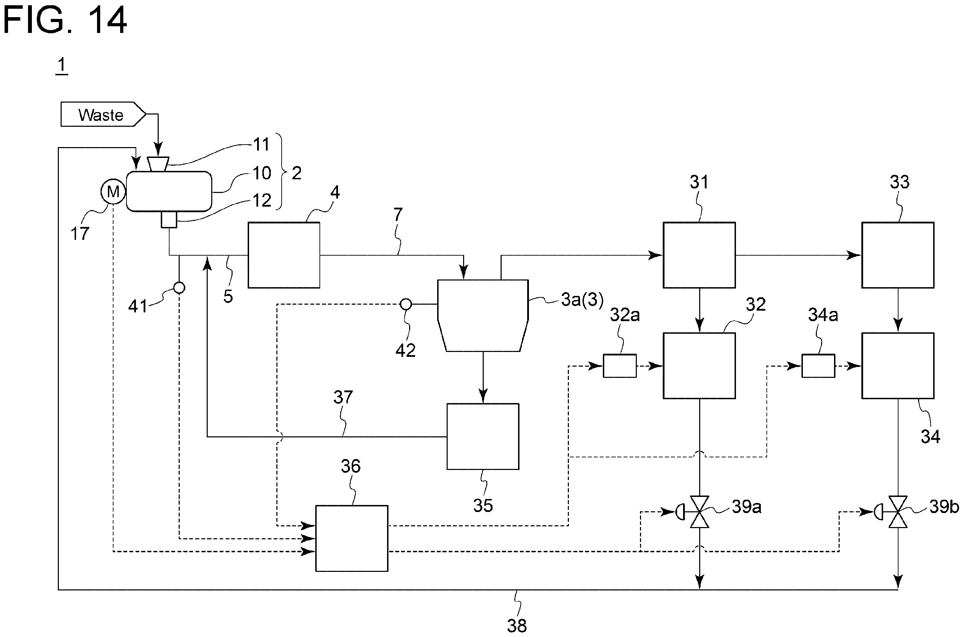

As shown in FIG. 14, the waste treatment system 1 according to Embodiment 5 of the present disclosure includes at least either of a volatile fatty acid sensor (VFA sensor) 41 for detecting a VFA concentration in the reformed material transferred in the reformed material transfer line 5 or a VFA sensor 42 for detecting a VFA concentration in the biogas fermentation tank 3a. The control device 36 is configured to receive a detected value of at least either of the VFA sensors 41 or 42. Other configurations are the same as those in Embodiment 4.

### <Operation of waste treatment system according to Embodiment 5 of present disclosure>

The operation in Embodiment 5 is basically the same as that in Embodiment 4. However, the operation in Embodiment 5 is different from that in Embodiment 4 in that the reaction conditions in the biogas fermentation tank 3a are detected based on the value detected by the VFA sensor 41, 42 as the indicator of the hydrolysis state of the waste in the reformer 2, the hydrolysis state in the reformer 2 is estimated from the detected reaction conditions, and based on the estimated hydrolysis state, the hydrolysis conditions of the next batch of waste in the reformer 2 are adjusted. Thus, the VFA sensor 41, 42 constitutes a detection device for detecting the indicator of the hydrolysis state of the waste in the reformer 2. Hereinafter, the operation of estimating the hydrolysis state of the waste in the reformer 2 will be described. In the following description, it is assumed that both the VFA sensors 41 and 42 are provided.

During operation of the waste treatment system 1, the VFA sensor 41 detects the VFA concentration in the reformed material flowing out from the reformer 2. The VFA sensor 42 detects the VFA concentration in the contents within the biogas fermentation tank 3a. The configurations of the VFA sensors 41 and 42 are not particularly limited, and thus any known sensor can be used to detect the VFA concentration. As an example, the configuration of a sensor that can be used as the VFA sensor 42 will be described. In the biogas fermentation tank 3a, a content circulation line for extracting a part of the contents to the outside and returning it to the biogas fermentation tank 3a again, a withdrawal line for withdrawing a part of the contents from the circulation line, and a dilution water feed line for feeding dilution water to the withdrawal line are provided, a probe for measuring the contents diluted with the dilution water is provided, and the contents diluted with the dilution water are irradiated with infrared rays from the probe and the transmitted light is received, thereby detecting the VFA concentration in the contents within the biogas fermentation tank 3a. The VFA sensor 41 for detecting the VFA concentration in the reformed material flowing out from the reformer 2 can detect the VFA concentration by using infrared rays.

A signal regarding the VFA concentration detected by the VFA sensor 41, 42 is transmitted from VFA sensor 41, 42 to the control device 36. An upper limit value and a lower limit value for the VFA concentration are set in advance in the control device 36, and it is determined whether the transmitted VFA concentration is between the upper limit value and the lower limit value. If the VFA concentration is less than the lower limit value, the reaction of methane fermentation slows down, and thus it is necessary to increase the VFA concentration. Specifically, for example, the temperature or the amount of steam supplied to the reformer 2 for hydrolysis of the next batch of waste is increased, the hydrolysis time is increased, or the total amount of VFA to be input to the biogas fermentation tank 3a is increased by increasing a supply amount or a supply frequency of a methane fermentation raw material per time. Conversely, if the VFA concentration is too high, VFA accumulates in the biogas fermentation tank 3a and causes rancidity. Thus, if the detected VFA concentration exceeds the upper limit value, for example, the temperature or the amount of steam supplied to the reformer 2 for hydrolysis of the next batch of waste is decreased, the hydrolysis time is decreased, or the supply amount or the supply frequency of the methane fermentation raw material is adjusted.

Thus, based on the VFA concentration in the reformed material obtained by hydrolyzing the waste or the VFA concentration in the contents within the biogas fermentation tank 3a, the hydrolysis state of the waste in the reformer 2 is estimated, and based on the estimated hydrolysis state, the hydrolysis conditions of the next batch of waste in the reformer 2 are adjusted. Whereby, the VFA concentration in the contents within the biogas fermentation tank 3a of the next batch can be maintained at conditions suitable for methane fermentation, making it possible to efficiently produce biogas by microbial reaction.

In Embodiment 5, the VFA sensor 41, 42 is permanently installed. However, the reformed material or the contents within the biogas fermentation tank 3a may be sampled to analyze the VFA concentration contained therein, and the hydrolysis state may be calculated from data of the analysis result.

### (Embodiment 6)

Next, the waste treatment system according to Embodiment 6 will be described. Relative to Embodiments 1 to 5, the waste treatment system according to Embodiment 6 modifies the operation of detecting the reaction conditions in the microbial reactor 3. Hereinafter, a configuration obtained by modifying the operation of detecting the reaction conditions in the microbial reactor 3 relative to Embodiment 5 will be described. However, Embodiment 6 may be configured by modifying the operation of detecting the reaction conditions in the microbial reactor 3 relative to any of Embodiments 1 to 4. In Embodiment 6, the same constituent elements as those in Embodiment 5 are associated with the same reference characters and not described again in detail.

### <Configuration of waste treatment system according to Embodiment 6 of present disclosure>

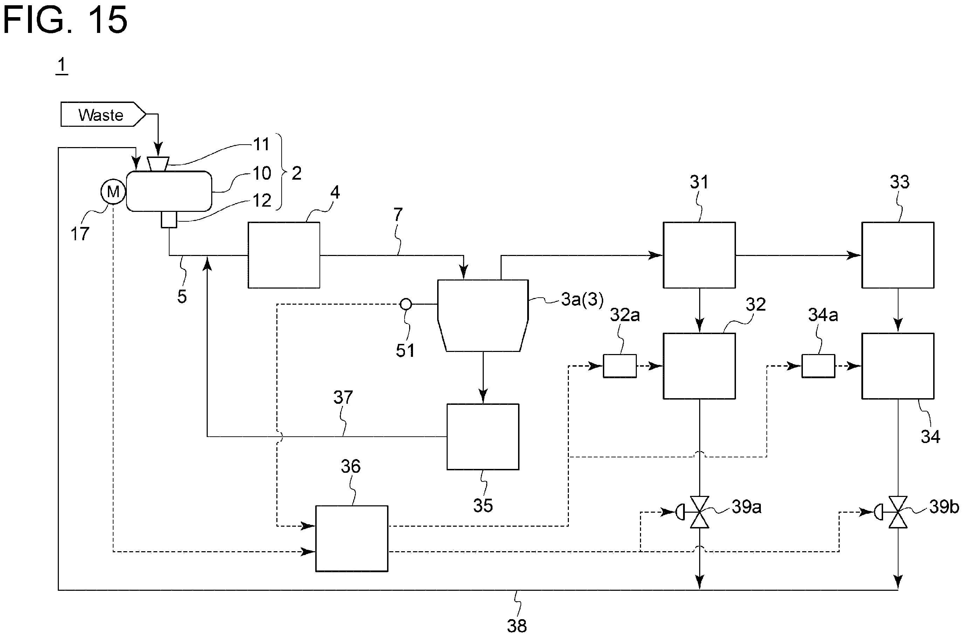

As shown in FIG. 15, the waste treatment system 1 according to Embodiment 6 of the present disclosure is provided with a fermentation inhibitor sensor 51 for detecting the concentration of the fermentation inhibitor such as melanoidin, furral, phenol within the biogas fermentation tank 3a as an indicator of the hydrolysis state of the waste in the reformer 2. The control device 36 is configured to receive a detected value of the fermentation inhibitor sensor 51. Other configurations are the same as those in Embodiment 5, except that the VFA sensor 41, 42 (see FIG. 14) is not provided.

### <Operation of waste treatment system according to Embodiment 6 of present disclosure>

The operation in Embodiment 6 is basically the same as that in Embodiment 5. However, the operation of detecting the reaction conditions in the biogas fermentation tank 3a is different. Hereinafter, the operation of detecting the reaction conditions in the biogas fermentation tank 3a will be described.

During operation of the waste treatment system 1, the fermentation inhibitor sensor 51 detects the concentration of the fermentation inhibitor in the contents within the biogas fermentation tank 3a. The configuration of the fermentation inhibitor sensor 51 is not particularly limited, and thus any known sensor can be used to detect the concentration of the fermentation inhibitor. As an example, the configuration of a sensor that can be used as the fermentation inhibitor sensor 51 will be described. In the biogas fermentation tank 3a, a content circulation line for extracting a part of the contents to the outside and returning it to the biogas fermentation tank 3a again, a withdrawal line for withdrawing a part of the contents from the circulation line, and a dilution water feed line for feeding dilution water to the withdrawal line are provided, a probe for measuring the contents diluted with the dilution water is provided, and the contents diluted with the dilution water are irradiated with ultraviolet rays from the probe and the transmitted light is received, thereby detecting the concentration of the fermentation inhibitor in the contents within the biogas fermentation tank 3a.

A signal regarding the concentration of the fermentation inhibitor detected by the fermentation inhibitor sensor 51 is transmitted from the fermentation inhibitor sensor 51 to the control device 36. An upper limit value for the concentration of the fermentation inhibitor is set in advance in the control device 36, and it is determined whether the concentration of the transmitted fermentation inhibitor is not greater than the upper limit value. If the concentration of the fermentation inhibitor exceeds the upper limit value, methane fermentation is inhibited and the reaction slows down, and thus it is necessary to increase the concentration of the fermentation inhibitor. Specifically, the temperature of steam or the supply amount of steam supplied to the reformer 2 for hydrolysis of the next batch of waste is decreased.

In Embodiment 6, since the hydrolysis state of the waste in the reformer 2 is estimated based on the concentration of the fermentation inhibitor detected by the fermentation inhibitor sensor 51, the fermentation inhibitor sensor 51 constitutes a detection device for detecting the hydrolysis state of the waste in the reformer 2.

Thus, based on the concentration of the fermentation inhibitor in the contents within the biogas fermentation tank 3a, the hydrolysis state of the waste in the reformer 2 is estimated, and based on the estimated hydrolysis state, the hydrolysis conditions of the next batch of waste in the reformer 2 are adjusted. Whereby, the concentration of the fermentation inhibitor in the contents within the biogas fermentation tank 3a of the next batch can be decreased, making it possible to efficiently produce biogas by microbial reaction.

### <Modified example of waste treatment system according to Embodiment 6 of present disclosure>

In Embodiment 6, the hydrolysis conditions of the next batch in the reformer 2 are adjusted based on the concentration of the fermentation inhibitor in the contents within the biogas fermentation tank 3a. However, the present invention is not limited to this mode. For example, in a case where the composition of each batch of waste hydrolyzed in the reformer 2 changes significantly, it is difficult to adjust to appropriate reaction conditions even if the hydrolysis conditions of the next batch are adjusted based on the reaction conditions in the biogas fermentation tank 3a of the previous batch.

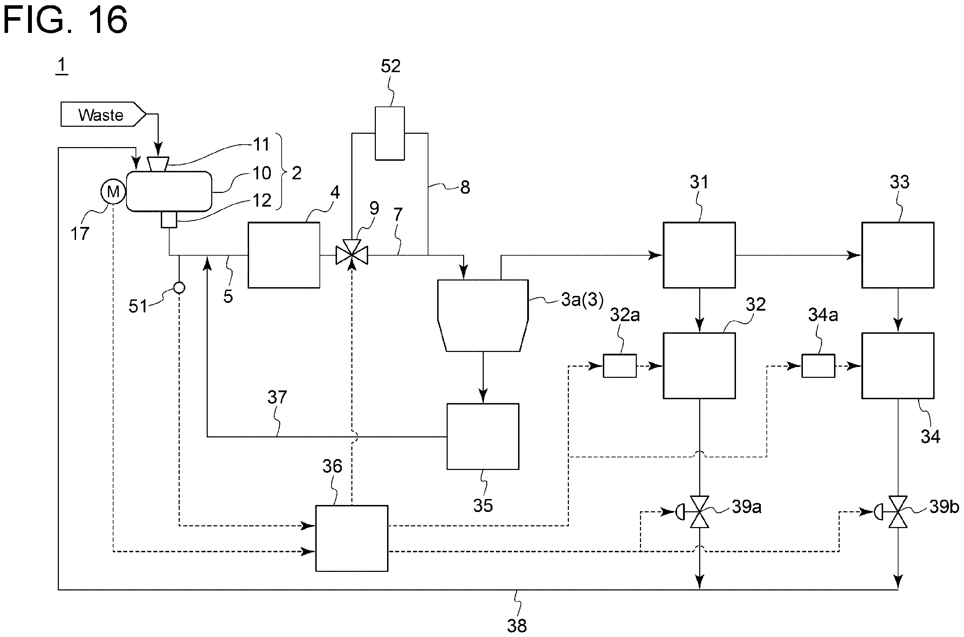

In such a case, as shown in FIG. 16, the fermentation inhibitor sensor 51 may be provided on the reformed material transfer line 5 such that the concentration of the fermentation inhibitor in the reformed material flowing out from the reformer 2 can be detected, and further, a detour line 8 may be provided which branches off from the reformed material transfer line 7 through which the separator 4 and the biogas fermentation tank 3a communicate with each other, and joins the reformed material transfer line 7 again downstream of the branch point, and at least one bypass tank 52 may be provided on the detour line 8. The switching device 9 may be provided at an upstream end of the detour line 8 in order to supply the reformed material flowing out from the separator 4 to either the bypass tank 52 or the biogas fermentation tank 3a.

In the modified example, if the detected value by the fermentation inhibitor sensor 51 exceeds the upper limit value, the control device 36 switches the switching device 9 such that the reformed material is not supplied to the biogas fermentation tank 3a but is stored in the bypass tank 52. If the concentration of the fermentation inhibitor in the reformed material for hydrolysis after the next batch becomes not greater than the upper limit value, the reformed material is gradually mixed with the reformed material which has a relatively high concentration of the fermentation inhibitor stored in the bypass tank 52 and is supplied to the biogas fermentation tank 3a. Thus, the concentration of the fermentation inhibitor in the contents within the biogas fermentation tank 3a can be suppressed to not greater than the upper limit value, making it possible to efficiently produce biogas by microbial reaction.

In Embodiment 6, the fermentation inhibitor sensor 51 is permanently installed. However, the reformed material or the contents within the biogas fermentation tank 3a may be sampled to analyze the concentration of the fermentation inhibitor contained therein, and the hydrolysis state may be calculated from data of the analysis result.

In Embodiment 6, the control device 36 may learn a correspondence relationship among the property of the waste, the hydrolysis conditions, and the hydrolysis state, and adjust the hydrolysis conditions based on a learned model constructed as a result of the learning.

In Embodiments 5 and 6, the VFA sensor 41, 42 and the fermentation inhibitor sensor 51 each serving as the detection device are described as the examples. However, the present invention is not limited thereto. The detection device may be a sensor for detecting the amount of a gas produced in the microbial reactor 3, the VFA concentration in the gas, the pH, electrical conductivity, alkalinity, ammonia concentration, bacterial cell concentration, etc. of the contents within the microbial reactor 3. Further, the detection device may be a sensor for detecting pH, electrical conductivity, chromaticity, inhibitor concentration, VFA concentration, ammonia concentration, particle size distribution, etc. during hydrolysis. By calculating an indicator with a function using these detected values, it is possible to estimate the hydrolysis state of the waste in the reformer 2.

In Embodiments 1 to 6, the reformer 2 hydrolyzes the waste in a batch manner. However, the reformer 2 may hydrolyze the waste in a flow manner. Note that, in the reformer for performing hydrolysis in a flow manner, the waste is given fluidity in order to supply the waste to the reformer, and, for example, a method for decreasing the diameter of the waste and supplying the waste as slurry supplied with a large amount of water is considered. In this case, extra energy, such as energy for decreasing the diameter of the waste or energy for increasing the temperature of the water in the slurry during hydrolysis, is required. By contrast, in the case of the reformer for performing hydrolysis in a batch manner, the diameter of the waste need not be decreased but the waste can be supplied into the reformer as it is, making it possible to eliminate the need for the above-described extra energy required in the reformer for performing hydrolysis in a flow manner.

The contents described in the above embodiments would be understood as follows, for instance.
[1] A waste treatment system according to one aspect includes: at least one reformer (2) for hydrolyzing waste; and a microbial reactor (3) for microbially degrading a reformed material containing at least a solid among the waste hydrolyzed in the at least one reformer (2).

According to the waste treatment system of the present disclosure, since the valuables can be produced by degrading the waste, which is hydrolyzed in the reformer, in the microbial reactor without solid-liquid separation, it is possible to treat even the waste with low moisture content (preferably, not greater than 70%) at low cost.

[2] A waste treatment system according to another aspect is the waste treatment system of [1], wherein the at least one reformer (2) includes: a housing (10) for receiving the waste; an input port (11) for inputting the waste to the housing (10); a discharge port (12) for discharging the reformed material from the housing (10); and opening and closing valves (18, 19) for opening and closing the input port (11) and the discharge port (12), respectively, and wherein the waste within the housing (10) is hydrolyzed in a batch manner while the housing (10) is sealed by closing each of the opening and closing valves (18, 19).

In the reformer for performing hydrolysis in a flow manner, the waste needs to be given fluidity in order to supply the waste to the reformer, and, for example, it is necessary to decrease the diameter of the waste and supply the waste as slurry supplied with a large amount of water. In this case, extra energy, such as energy for decreasing the diameter of the waste or energy for increasing the temperature of the water in the slurry during hydrolysis, is required. By contrast, with the above configuration [2], since hydrolysis is performed in a batch manner, the diameter of the waste need not be decreased but the waste can be supplied into the reformer as it is, making it possible to eliminate the need for the above-described extra energy required in the reformer for performing hydrolysis in a flow manner.

[3] A waste treatment system according to another aspect is the waste treatment system of [1] or [2], wherein the at least one reformer (2) includes a housing (10) for receiving the waste, and wherein the at least one reformer (2) is configured to supply steam into the housing (10) to heat and hydrolyze the waste within the housing (10) with the steam.

With such configuration, it is possible to easily adjust the hydrolysis condition by adjusting the temperature, pressure, supply amount, etc. of the steam.

[4] A waste treatment system according to another aspect is the waste treatment system of [1] or [2], wherein the at least one reformer (2) includes a housing (10) for receiving the waste, and wherein the housing (10) includes a heating part (jacket 15) for hydrolyzing the waste within the housing (10) by heating moisture contained in the waste while avoiding contact with the waste.

With such configuration, it is possible to easily adjust the hydrolysis condition by adjusting the temperature, pressure, supply amount, etc. of the steam.

[5] A waste treatment system according to another aspect is the waste treatment system of [1] to [4], wherein, in the at least one reformer (2), hydrolysis is performed at temperatures between normal temperature and 250°C and at pressures between atmospheric pressure and 40 atmospheres.

With such configuration, it is possible to set such that a cell fluid flows out from a cell contained in kitchen waste. Consequently, the kitchen waste is made finer, and a polymer component is subjected to degradation. In addition, volatile fatty acid (VFA) such as acetic acid is increased.

[6] A waste treatment system according to another aspect is the waste treatment system of [1] to [5], including: a first reformer (2a) and a second reformer (2b) each serving as the at least one reformer (2); and a solid-liquid separator (70) for performing solid-liquid separation on a treated object which is the waste hydrolyzed in the first reformer (2a). The second reformer (2b) hydrolyzes only a solid separated by the solid-liquid separator (70).

With such configuration, by solid-liquid separation, a nitrogen compound, such as protein, that causes production of melanoidin can be separated to a liquid side, making it possible to suppress production of melanoidin in hydrolysis in the second reformer. Inflow of melanoidin into a methane fermentation tank serving as the microbial reactor inhibits methane fermentation. Thus, by performing solid-liquid separation on the reformed material of the first reformer to transfer only the solid obtained by solid-liquid separation to the second reformer, it is possible to reduce a risk of inhibiting methane fermentation in the methane fermentation tank.

[7] A waste treatment system according to another aspect is the waste treatment system of [1] to [6], wherein the microbial reactor (3) includes at least one of: a biogas fermentation tank (3a) for producing biogas; a saccharification tank for producing sugar from carbohydrate; and a composting device for producing compost.

With such configuration, it is possible to produce one type or not less than two types of desired valuables.

[8] A waste treatment system according to another aspect is the waste treatment system of [1] to [7], further including: a separator (4) between the at least one reformer (2) and the microbial reactor (3), the separator (4) being configured to separate a reaction-unsuitable substance, which is unsuitable for the microbial degradation in the microbial reactor (3), from the reformed material.

With such configuration, since the reaction-unsuitable substance is separated from the reformed material by the separator, it is possible to reduce the amount of the reaction-unsuitable substance supplied to the microbial reactor. As a result, it is possible to reduce the risk of inhibiting the microbial reaction in the microbial reactor, and to efficiently produce the valuables by the microbial reaction.

[9] A waste treatment system according to another aspect is the waste treatment system of [8], including a moisture adjusting device for adjusting a moisture content of the reformed material.

With such configuration, since it is possible to adjust the TS of the reformed material flowing out from the reformer, it is possible to successfully separate the reaction-unsuitable material in the separator.

[10] A waste treatment system according to another aspect is the waste treatment system of [9], wherein the moisture adjusting device is a drying device (71) for reducing the moisture content of the reformed material.

With such configuration, when treating something like sludge with a relatively high moisture content, since the TS of the reformed material flowing out from the reformer is low, the reaction-unsuitable substance may not successfully be separated in the separator. By contrast, with the above configuration [10], since it is possible to adjust the TS of the reformed material by reducing the moisture content of the reformed material by the drying device, it is possible to successfully separate the reaction-unsuitable material in the separator.

[11] A waste treatment system according to another aspect is the waste treatment system of [9], wherein the moisture adjusting device is a washing device for washing a reaction-suitable substance adhering to the reaction-unsuitable substance.

With such configuration, the reaction-suitable substance can be supplied to the microbial reactor after the content of the fermentation inhibitor in the reaction-suitable substance is reduced, making it possible to reduce the risk of inhibiting the reaction in the microbial reactor. Further, if the moisture adjusting device is used as a washing device for washing the reaction-unsuitable substance which is the large particle size component separated in the separator, it is possible to recover the small particle size component adhering to the reaction-unsuitable substance as the reaction-suitable substance, and it is possible to improve the recovery rate of the reaction-suitable substance.

[12] A waste treatment system according to another aspect is the waste treatment system of [8] to [10], including: a dehydrator for dehydrating a residue of the microbial reactor (3); and a water injection pipe for feeding water dehydrated by the dehydrator to at least one of the reformed material before flowing into the separator (4), inside of the reformer (2), or inside of the microbial reactor (3).

With such configuration, since water, where a residue of the microbial reactor is dehydrated, contains ammonia, the reformed material can also be replenished with a nitrogen-containing substance if the nitrogen content in the waste within the reformer or in the contents within the microbial reactor is low.

[13] A waste treatment system according to another aspect is the waste treatment system of [8] to [12], wherein the separator (4) is a screen for separating the reformed material into a large particle size component and a small particle size component having a smaller particle size than the large particle size component, and the large particle size component is the reaction-unsuitable substance.

If the microbial reactor is the composting device, it is necessary to keep the moisture content low. Thus, as in the above configuration [12], separation by the screen is suitable.

[14] A waste treatment system according to still another aspect is the waste treatment system of any of [1] to [13], further including: a detection device (control device 36) for detecting an indicator of a hydrolysis state of the waste in the at least one reformer (2); an adjustment device (combustion boiler controller 32a/exhaust gas boiler controller 34a/steam supply amount adjustment valve 39a, 39b) for adjusting a hydrolysis condition of the waste in the at least one reformer (2); and a control device (36). The control device (36) estimates the hydrolysis state of the waste based on the indicator detected by the detection device (control device 36), and operates the adjustment device (32a/34a/39a, 39b) to adjust the hydrolysis condition of the waste based on the estimated hydrolysis state.

With such configuration, since the reformed material on the conditions suitable for microbial reaction can be supplied to the microbial reactor, it is possible to efficiently produce valuable by microbial reaction.

[15] A waste treatment system according to another aspect is the waste treatment system of [14], wherein the at least one reformer (2) includes: an agitator (16) for agitating waste; and a motor (17) for driving the agitator (16). The detection device (36) detects a torque of the motor (17) as the indicator. The control device (36) estimates the hydrolysis state based on the torque of the motor (17).

With such configuration, since the reformed material on the conditions suitable for microbial reaction can be supplied to the microbial reactor, it is possible to efficiently produce valuable by microbial reaction.

[16] A waste treatment system according to another aspect is the waste treatment system of [14], wherein the at least one reformer (2) includes: an agitator (16) for agitating waste; and a motor (17) for driving the agitator (16). The detection device (36) detects a current value of the motor (17) as the indicator. The control device (36) estimates the hydrolysis state based on the current value of the motor (17).

With such configuration, since the reformed material on the conditions suitable for microbial reaction can be supplied to the microbial reactor, it is possible to efficiently produce valuable by microbial reaction.

[17] A waste treatment system according to another aspect is the waste treatment system of [14], wherein the detection device detects information about a property of the waste as the indicator, and wherein the control device estimates the hydrolysis state based on the property of the waste.

With such configuration, since the reformed material on the conditions suitable for microbial reaction can be supplied to the microbial reactor, it is possible to efficiently produce valuable by microbial reaction.

[18] A waste treatment system according to another aspect is the waste treatment system of [17], wherein the control device learns a correspondence relationship among the property of the waste, the hydrolysis condition, and the hydrolysis state, and adjusts the hydrolysis condition based on a learned model constructed as a result of the learning.

With such configuration, compared to the above [17], it is possible to more efficiently produce valuable by microbial reaction.

[19] A waste treatment system according to another aspect is the waste treatment system of [14] to [18], wherein the waste is heated and hydrolyzed with steam supplied to the at least one reformer, and wherein the adjustment device adjusts at least one of a temperature, a pressure, and a supply amount of the steam.

With such configuration, it is possible to easily adjust the hydrolysis condition.

[20] A waste treatment method according to one aspect includes: a step of hydrolyzing waste; and a step of microbially degrading a reformed material containing at least a solid among the hydrolyzed waste.

According to the waste treatment method of the present disclosure, since the valuables can be produced by microbially degrading the hydrolyzed waste without solid-liquid separation, it is possible to treat even the waste with low moisture content at low cost.

### Reference Signs List

- 1: Waste treatment system
- 2: Reformer
- 2a: First reformer
- 2b: Second reformer
- 3: Microbial reactor
- 4: Separator
- 10: Housing
- 11: Input port
- 12: Discharge port
- 15: Jacket (heating part)
- 16: Agitator
- 17: Motor
- 18: Opening and closing valve
- 19: Opening and closing valve
- 32a: Combustion boiler controller (adjustment device)
- 34a: Exhaust gas boiler controller (adjustment device)
- 35: Dehydrator
- 36: Control device (detection device)
- 37: Water injection pipe (moisture adjusting device)
- 39a: Steam supply amount adjustment valve (adjustment device)
- 39b: Steam supply amount adjustment valve (adjustment device)
- 41: VFA sensor (detection device)
- 42: VFA sensor (detection device)
- 51: Fermentation inhibitor sensor (detection device)
- 70: Solid-liquid separator
- 71: Drying device (moisture adjusting device)

## Claims

1. A waste treatment system, comprising:
at least one reformer for hydrolyzing waste; and
a microbial reactor for microbially degrading a reformed material containing at least a solid among the waste hydrolyzed in the at least one reformer.

2. The waste treatment system according to claim 1,
wherein the at least one reformer includes:
a housing for receiving the waste;
an input port for inputting the waste to the housing;
a discharge port for discharging the reformed material from the housing; and
opening and closing valves for opening and closing the input port and the discharge port, respectively, and
wherein the waste within the housing is hydrolyzed in a batch manner while the housing is sealed by closing each of the opening and closing valves.

3. The waste treatment system according to claim 1 or 2,
wherein the at least one reformer includes a housing for receiving the waste, and
wherein the at least one reformer is configured to supply steam into the housing to heat and hydrolyze the waste within the housing with the steam.

4. The waste treatment system according to claim 1 or 2,
wherein the at least one reformer includes a housing for receiving the waste, and
wherein the housing includes a heating part for hydrolyzing the waste within the housing by heating moisture contained in the waste while avoiding contact with the waste.

5. The waste treatment system according to any one of claims 1 to 4,
wherein, in the at least one reformer, hydrolysis is performed at temperatures between normal temperature and 250°C and at pressures between atmospheric pressure and 40 atmospheres.

6. The waste treatment system according to any one of claims 1 to 5, comprising:
a first reformer and a second reformer each serving as the at least one reformer; and
a solid-liquid separator for performing solid-liquid separation on a treated object which is the waste hydrolyzed in the first reformer,
wherein the second reformer hydrolyzes only a solid separated by the solid-liquid separator.

7. The waste treatment system according to any one of claims 1 to 6,
wherein the microbial reactor includes at least one of:
a biogas fermentation tank for producing biogas;
a saccharification tank for producing sugar from carbohydrate; and
a composting device for producing compost.

8. The waste treatment system according to any one of claims 1 to 7, further comprising:
a separator between the at least one reformer and the microbial reactor, the separator being configured to separate a reaction-unsuitable substance, which is unsuitable for the microbial degradation in the microbial reactor, from the reformed material.

9. The waste treatment system according to claim 8, comprising a moisture adjusting device for adjusting a moisture content of the reformed material.

10. The waste treatment system according to claim 9,
wherein the moisture adjusting device is a drying device for reducing the moisture content of the reformed material.

11. The waste treatment system according to claim 9,
wherein the moisture adjusting device is a washing device for washing a reaction-suitable substance adhering to the reaction-unsuitable substance.

12. The waste treatment system according to any one of claims 8 to 10, comprising:
a dehydrator for dehydrating a residue of the microbial reactor; and
a water injection pipe for feeding water dehydrated by the dehydrator to at least one of the reformed material before flowing into the separator, inside of the reformer, or inside of the microbial reactor.

13. The waste treatment system according to any one of claims 8 to 12,
wherein the separator is a screen for separating the reformed material into a large particle size component and a small particle size component having a smaller particle size than the large particle size component, and the large particle size component is the reaction-unsuitable substance.

14. The waste treatment system according to any one of claims 1 to 13, further comprising:
a detection device for detecting an indicator of a hydrolysis state of the waste in the at least one reformer;
an adjustment device for adjusting a hydrolysis condition of the waste in the at least one reformer; and
a control device,
wherein the control device estimates the hydrolysis state of the waste based on the indicator detected by the detection device, and operates the adjustment device to adjust the hydrolysis condition of the waste based on the estimated hydrolysis state.

15. The waste treatment system according to claim 14,
wherein the at least one reformer includes:
an agitator for agitating waste; and
a motor for driving the agitator,
wherein the detection device detects a torque of the motor as the indicator, and
wherein the control device estimates the hydrolysis state based on the torque of the motor.

16. The waste treatment system according to claim 14,
wherein the at least one reformer includes:
an agitator for agitating waste; and
a motor for driving the agitator,
wherein the detection device detects a current value of the motor as the indicator, and
wherein the control device estimates the hydrolysis state based on the current value of the motor.

17. The waste treatment system according to claim 14,
wherein the detection device detects information about a property of the waste as the indicator, and
wherein the control device estimates the hydrolysis state based on the property of the waste.

18. The waste treatment system according to claim 17,
wherein the control device learns a correspondence relationship among the property of the waste, the hydrolysis condition, and the hydrolysis state, and adjusts the hydrolysis condition based on a learned model constructed as a result of the learning.

19. The waste treatment system according to any one of claims 14 to 18,
wherein the waste is heated and hydrolyzed with steam supplied to the at least one reformer, and
wherein the adjustment device adjusts at least one of a temperature, a pressure, and a supply amount of the steam.

20. A waste treatment method, comprising:
a step of hydrolyzing waste; and
a step of microbially degrading a reformed material containing at least a solid among the hydrolyzed waste.
